# EUROPEAN PATENT SPECIFICATION

(11) **EP 2 085 020 B1**
(45) Date of publication and mention of the grant of the patent: **28.09.2011**
(21) Application number: 09001187.5
(22) Date of filing: 28.01.2009
(51) Int. Cl.: A61B 1/05

(54) **Capsule endoscope and capsule endoscope system**
Kapselendoskop und Kapselendoskopsystem
Endoscope à capsule et système d'endoscope à capsule

(30) Priority: 29.01.2008 JP 2008017070
(43) Date of publication of application: 05.08.2009
(73) Proprietor: FUJIFILM Corporation, Minato-ku Tokyo (JP)
(72) Inventor: Nishino, Naoyuki, Kanagawa (JP)
(74) Representative: Klunker . Schmitt-Nilson . Hirsch

(56) References cited:
- EP-A- 1 510 169
- EP-A- 1 800 592
- EP-A- 1 857 036
- US-A1- 2007 225 560

## Description

### FIELD OF THE INVENTION

The present invention relates to a capsule endoscope for shooting images inside a body of a patient and a capsule endoscope system.

### BACKGROUND OF THE INVENTION

A capsule endoscope incorporating an image pickup section including a taking lens and an image sensor, and a light source in an extremely small capsule is known. In addition to the image pickup section and the light source, the capsule endoscope includes a signal processor that performs gain control, denoising and A/D conversion of a pickup signal (analogue signal of each of R, G and B colors) output from the image sensor, an antenna that transmits radio waves, on which the pickup image that has been converted into digital data is superimposed, to a receiver provided outside the patient' s body, and electric components such as a battery for supplying electric power to each part.

Even among the capsule endoscopes of same model, they have individual difference in color of the pickup image depending on the type of the light source or ununiformity in luminescent color. Therefore, color calibration is performed in an adjustment process at the time of manufacture. A correction value obtained through the color calibration is set as a gain control value of the pickup signal.

In addition, since transmit frequency of antenna varies from country to country, the transmit frequency is set to correspond with the destination country during the adjustment process. The electric components thus adjusted are wired and incorporated in the capsule in an assembly process, and thereby completing a capsule endoscope.

However, variance in attachment position of the taking lens during the assembly may cause the difference in color of the pickup image. Therefore, the individual difference of the completed product cannot be compensated only by the above-described color calibration. In view of this, a capsule endoscope cited in Japanese Patent Laid-open Publication No. 2005-021651 includes a test chart for color calibration in a package of a completed product, and a test image obtained by shooting the test chart is sent to a receiver before use, so that the receiver can calculate an image correction value based on the test image. The pickup image sent from the capsule endoscope is corrected at the receiver side based on the calculated image correction value, and therefore the individual difference of the completed product regarding the color of the pickup image can be compensated.

In addition, a capsule endoscope capable of bidirectional communication by incorporating a receiving antenna inside a capsule, independently from a transmitting antenna, for receiving a setting command of transmit frequency which enables changing of the transmit frequency of the capsule endoscope as a completed product is disclosed in Japanese Patent Application Laid-open Publication No. 2007-089892. For this configuration, the setting of the transmit frequency depending upon the destination country need not be performed in the adjustment process which is performed before the assembly process. Therefore, neither different production lines nor adjustment of the production amount depending on the destination country is needed, which enhances the productivity.

When such color calibration and change of transmit frequency can be performed to the completed product, it is advantageous not only for the maker but also for the user because customization based on the user's needs becomes possible.

However, although the invention disclosed in the Japanese Patent Laid-open Publication No. 2005-021651 enables the color calibration on the completed product, other setting changes, such as the change of transmit frequency, cannot be made. Moreover, the above-described color calibration is performed by correcting the color of the pickup image which has been converted into the digital data at the receiver side based on the correction value obtained from the test image. According to this method, desired color correction cannot be made due to the influence of the analog signal noise during the conversion of the pickup signal into the digital data. To perform the desired correction, it is preferable to adjust the color by adding gain to the analog signal which has just been output from the image sensor.

The capsule endoscope disclosed in the Japanese Patent Laid-open Publication No. 2007-089892 enables bidirectional communication by providing two antennas or two communication circuits (transmitting circuit and receiving circuit), and thereby the setting changes, such as not only the change of transmit frequency but also the change of gain control value and the like, can be made. However, the provision of two antennas or two communication circuits causes increase in cost and number of parts, and further causing the enlargement of the capsule since the antennas are relatively large compared to other electric components and the antennas require larger capacity of the battery so that enough power is supplied to the antennas.

In accordance with the preamble of claim 1, EP-A-1 800 592 discloses a capsule endoscope which transmits image data to an external receiving device, which includes an image processing circuit which acquires a white balance(WB)-correction value used for a white-balance correction.

### SUMMARY OF THE INVENTION

An object of the present invention is to provide a capsule endoscope capable of changing various settings thereof in a completed state and a capsule endoscope system.

In order to achieve the above and other objects, a capsule endoscope according to the present invention comprises the features of claim 1.

Preferred embodiments are defined by dependent claims.

The setting change command is preferably a geometric pattern such as a two-dimensional code, an optical signal such as an infrared ray signal, or a color pattern such as a color code.

The data based on the image pickup signal may be image pickup information or a response to the setting change command.

It is preferable that the capsule endoscope further includes a mode change section for switching between a setting change mode for receiving the setting change command and a normal shooting mode for shooting the region to be observed, based on a mode change command obtained by the image pickup section.

It is preferable that the mode change command is light including at least one of color, brightness and flicker not appearing when the region to be observed is illuminated with the illumination section.

It is preferable that the mode change section switches from the normal shooting mode to the setting change mode within a certain period of time after a power is turned on.

It is also possible that the capsule endoscope is activated under the setting change mode for receiving the setting change command when the power is turned on, and switches from the setting change mode to the normal shooting mode for shooting the region to be observed when a predetermined condition is satisfied.

The setting change section and the mode change section are realized by, for example, software.

The setting change command sets, for example, drive condition of the illumination section. The drive condition may include at least one of a drive current value and a light-up time of a light source constituting the illumination section.

The setting change command may set a frame rate for image shooting by the image pickup section, or at least one of a transmit frequency, a modulation method and an output value of the transmitting section.

A capsule endoscope system according to the present invention includes the features of claim 19.

According to the capsule endoscope and the capsule endoscope system of the present invention, the setting of at least one of the illumination section, the image pickup section and the transmitting section is changed according to the setting change command optically detectable by the image pickup section. Owing to this, various settings of the capsule endoscope can be changed in the completed state without causing cost increase or enlargement of the article.

### BRIEF DESCRIPTION OF THE DRAWINGS

One with ordinary skill in the art would easily understand the above-described objects and advantages of the present invention when the following detailed description is read with reference to the drawings attached hereto:
Figure 1 is a schematic diagram illustrating a configuration of a capsule endoscope system according to an embodiment of the present invention;
Figure 2 is a cross sectional view illustrating an internal configuration of a capsule endoscope;
Figure 3 is a block diagram illustrating an electrical configuration of the capsule endoscope;
Figure 4 is a block diagram illustrating an electrical configuration of a driver controlling a drive of a light source section;
Figure 5 is a block diagram illustrating a configuration of each part built in a CPU of the capsule endoscope;
Figure 6 is an explanatory view of a remote controller for mode change;
Figure 7 is an explanatory view illustrating an example of a setting value table;
Figure 8 is an explanatory view illustrating an example of a two-dimensional code (QR code);
Figure 9 is a schematic diagram illustrating a configuration of a command production device;
Figure 10 is an explanatory view illustrating an example of a setting window;
Figure 11 is a block diagram illustrating an electrical configuration of a work station;
Figure 12 is a flow chart showing an operation procedure at the time of changing settings of the capsule endoscope;
Figure 13 is a flow chart showing an operation procedure at the time of changing settings of a capsule endoscope according to another embodiment;
Figure 14 is an explanatory view illustrating an example of a color code; and
Figure 15 is an explanatory view of a remote controller for infrared ray transmission.

### DESCRIPTION OF THE PREFERRED EMBODIMENTS

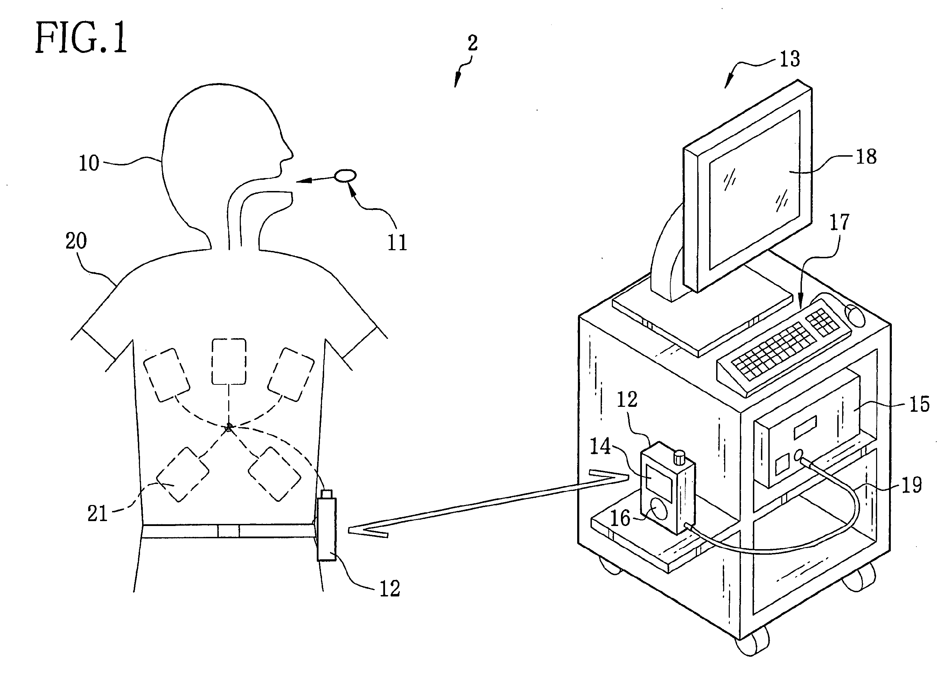

As shown in Fig. 1, a capsule endoscope system 2 includes a capsule endoscope (hereinafter abbreviated as CE) 11, a receiver 12, and a work station (hereinafter abbreviated as WS) 13. The capsule endoscope 11 is taken into a body through a mouth of a patient 10. The receiver 12 is attached to a belt of the patient 10. The WS 13 is used by a doctor to read an image obtained by the CE 11 for diagnosis.

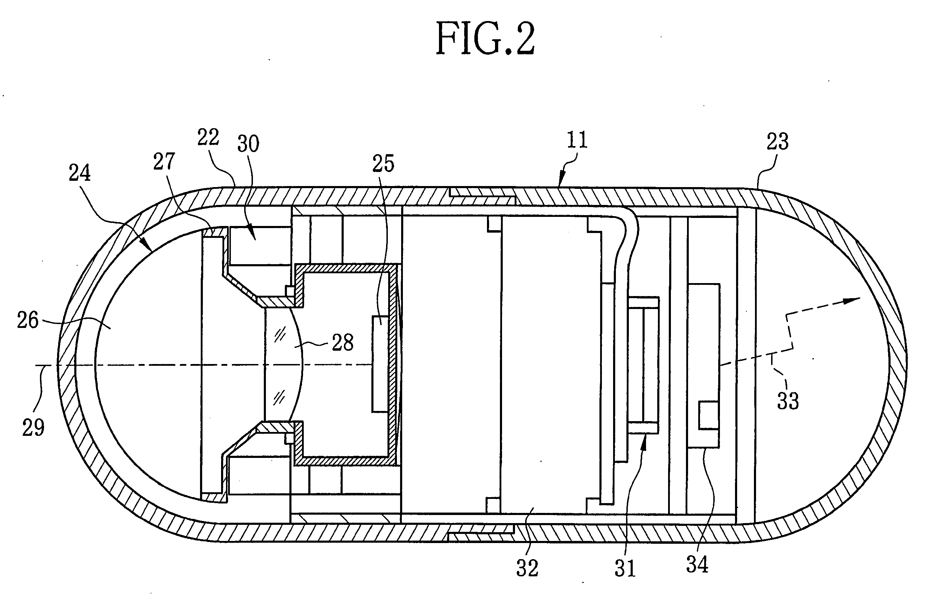

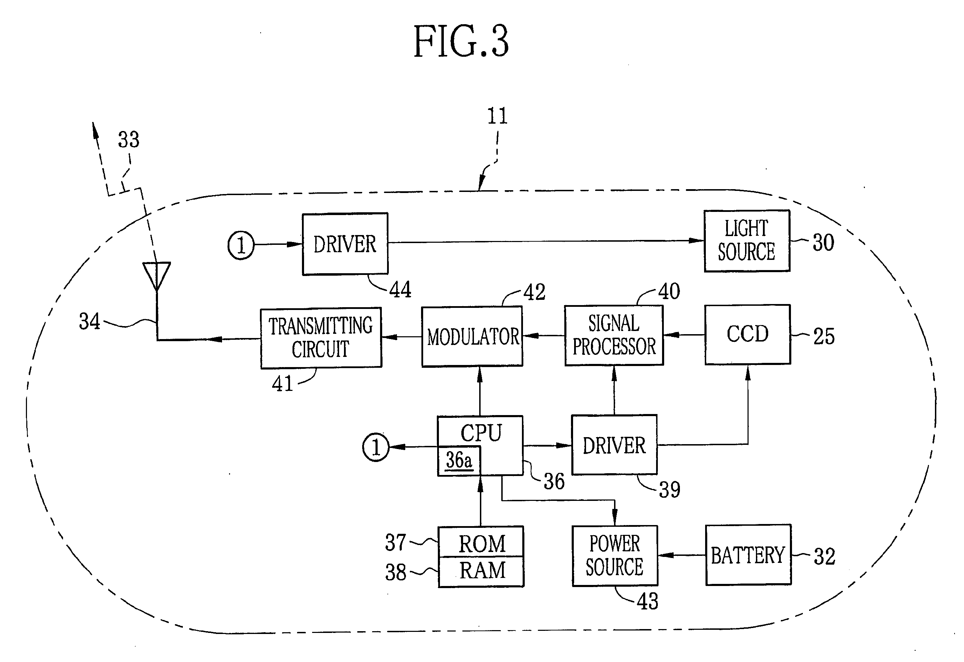

While passing through tracts in the human body, the CE 11 shoots images of inner wall surface of the tracts. The obtained image data is wirelessly transmitted to the receiver 12 via a radio wave 33 (see Figs. 2 and 3).

The receiver 12 includes a liquid crystal display 14 for displaying various setting screens, and an input panel 16 for performing various settings. The receiver 12 receives and stores image data wirelessly transmitted from the CE 11 via the radio wave 33.

The WS 13 includes a processor 15, a console 17 constituted of a key board and a mouse, and a monitor 18. The processor 15 is connected to the receiver 12 through a USB cable 19 (alternatively, wireless communication such as infrared communication), for example, and exchanges the data with the receiver 12. The processor 15 retrieves the image data from the receiver 12 during or after the endoscopy using the CE 11, and stores/manages the image data for each patient. In addition, the processor 15 produces an image for displaying from the image data, and displays the image on the monitor 18.

The radio wave 33 is transmitted/received between the CE 11 and the receiver 12 through a transmitting antenna 34 (see Figs. 2 and 3) provided in the CE 11 and plural receiving antennas 21 attached to a shield shirt 20 worn by the patient 10.

As shown in Fig. 2, the CE 11 includes a transparent front cover 22 and a rear cover 23 fitted into the front cover 22 to form a water-tight space. Each of the front cover 22 and the rear cover 23 has a tubular-shape whose front end or rear end is almost hemispheric.

The space formed by the front cover 22 and the rear cover 23 contains an image pickup section including an objective optical system 24 for collecting image light of a region to be observed and a CCD 25 for shooting the image of the region to be observed. The image light of the region to be observed entering through the objective optical system 24 is focused on a pickup surface of the CCD 25. The CCD 25 outputs a pickup signal of each pixel.

The objective optical system 24 includes a transparent convex optical dome 26 attached to the almost hemispherical front end of the front cover 22, a lens holder 27 narrowed toward its rear end and attached to the rear end of the optical dome 26, and a lens 28 fixed to the lens holder 27. The objective optical system 24 covers an imaging field with front viewing angle of 140° to 180°, for example, around an optical axis 29, and collects the image light of omnidirectional image of the region to be observed in the imaging field.

Inside the front cover 22 and the rear cover 23 there are contained not only the image pickup section but also a light source section 30 for emitting light to the region to be observed, an electrical circuit board 31 onto which a transmitting circuit 41 and a power source 43 (see Fig. 3) are mounted, a button cell battery 32, the transmitting antenna 34 for transmitting the radio wave 33, and the like.

As shown in Fig. 3, a CPU 36 controls all the operation of the CE 11 as a whole. A ROM 37 and a RAM 38 are connected to the CPU 36. Various programs and data for controlling the operation of the CE 11 are stored in the ROM 37. The CPU 36 retrieves necessary programs and data from the ROM 37 and expands the programs and data to the RAM 38 to sequentially process the retrieved programs.

A driver 39 and a signal processor 40 are connected to the CCD 25. The driver 39 controls the operation of the CCD 25 and the signal processor 40 based on a clock generator 36a incorporated in the CPU 36 such that image shooting is performed with a set frame rate. The signal processor 40 subjects the pickup signal outputted from the CCD 25 to corelated double sampling, amplification, and A/D conversion, and thereby converts the pickup signal to the digital image data. Then, the signal processor 40 subjects the converted image data to various kinds of image processing such as y conversion. Note that the amplification by the image processing section 40 is performed with gain set to correspond with the pixel of each of R, G and B colors. Each gain of RGB is referred to as R gain, G gain and B gain.

A transmitting circuit 41 is connected to the transmitting antenna 34 and a modulator 42. The modulator 42 modulates the digital image data, outputted from the signal processor 40, to the radio wave 33 of set frequency, and outputs the modulated radio wave 33 to the transmitting circuit 41. The transmitting circuit 41 subjects the radio wave 33 received from the modulator 42 to amplification and band-pass filtering, and then outputs the radio wave 33 to the transmitting antenna 34.

The power source 43 supplies the electric power of the battery 32 to respective components of the CE 11. A driver 44 controls the operation of the light source section 30 under the control of the CPU 36.

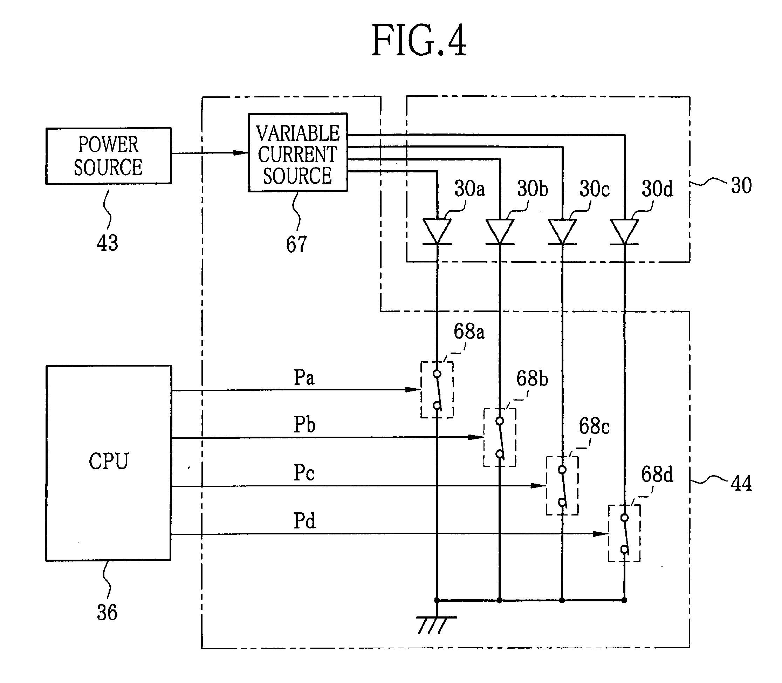

As shown in Fig. 4, the driver 44 has a variable current source 67 and four switching elements 68a, 68b, 68c and 68d. The variable current source 67 is connected to each input terminal of light sources 30a, 30b, 30c and 30d constituting the light source section 30. Owing to the electric power supplied from the power source 43, the variable current source 67 outputs drive current for turning on each light source 30a to 30d. The drive current is set to be lower than the rated current of each light source 30a to 30d, and the value of the drive current is controlled by the CPU 36.

The switching elements 68a to 68d are well-known semiconductor switching elements such as a FET. The output terminals of the light sources 30a to 30d are connected to the switching elements 68a to 68d, respectively. The other sides of the switching elements 68a to 68d opposite to the sides connected to the light sources 30a to 30d are connected to ground.

The switching elements 68a to 68d are turned on when pulse signals Pa, Pb, Pc and Pd from the CPU 36 are at a high level and turned off when the pulse signals Pa to Pd are at a low level. The light sources 30a to 30d are respectively lit up when the switching elements 68a to 68d are turned on, whereas the light sources 30a to 30d are respectively lit off when the switching elements 68a to 68d are turned off. The time while the pulse signals Pa to Pd are at the high level, that is, while the light sources 30a to 30d are lit up is time from the initiation of exposure of the CCD 25 until the exposure amount reaches the determined value.

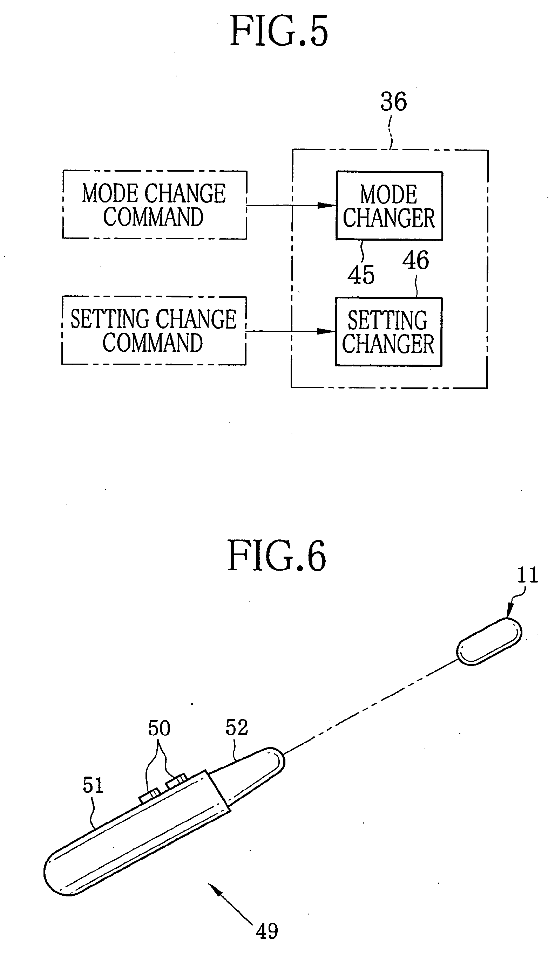

When the program (software) stored in the ROM 37 is activated as the electric power is supplied from the power source 43 to the respective components of the CE 11, a mode changer 45 and a setting changer 46 are built in the CPU 36 as shown in Fig. 5.

The mode changer 45 performs switching between a setting change mode for changing settings of the CE 11 and a normal shooting mode for performing normal image shooting. At an initial state when the CE 11 is turned on, the CE 11 is set in the normal shooting mode.

A mode change command is of light having at least one of color, brightness and flicker (timing of flicker) which will not appear when the inside of the body of the patient 10 is illuminated with the light source 30 so that the mode is not unintentionally switched inside the body of the patient 10. When the inside of the patient 10's body is illuminated with the light source 30, hemoglobin contained in red blood cells in the blood flowing through the body strongly absorbs light at wavelengths near 540 nm and light at wavelengths near 578 nm, and thereby light of reddish-purple or purple may appear. Therefore, the color which will not appear is, for example, in color of green, blue, yellow or the like. When any of such colors makes up predetermined percentage or more (for example, 80% or more) of the pickup image, the color is recognized as the mode change command. The brightness of light as the mode change command is, for example, several or several tens as much brighter than the brightness of reflection of the illumination light of the light source 30 on the region to be observed. The flicker of light as the mode change command has the timing of flicker which is far different from that of the illumination light, that is, for example, flickering every few seconds.

The mode change command is produced by, for example, a remote controller 49 for mode change as shown Fig. 6. The remote controller 49 is constituted of a holder 51 having operation buttons 50, and an illuminator 52 disposed at a front end of the holder 51. The remote controller 49 emits the light as the mode change command from the illuminator 52 when the operation button 50 is depressed.

The CE 11 picks up the light as the mode change command from the remote controller 49 with the CCD 25 and the mode changer 45 receives the image data obtained by picking up the mode change command. Owing to this, the mode changeover between the normal shooting mode and the setting change mode is performed. In order to distinguish that the mode has been switched, the light sources 30a to 30d are lit up under the normal shooting mode, whereas the light sources 30a to 30d are turned off under the setting change mode. Note that the light sources 30a to 30d are lit up even under the setting change mode at the time of adjusting the color of the pickup image, the drive current value and/or light-up time of the light sources 30a to 30d, and the like. The mode change command for switching from the normal shooting mode to the setting change mode and the mode change command for switching from the setting change mode to the normal shooting mode may be common like this embodiment, or may be different from each other.

The switchover from the normal shooting mode to the setting change mode is performed in an adjustment process of the manufacture of the CE 11, that is, after the respective components constituting the CE 11 are incorporated in the capsule and the CE 11 is completed as a final product. In the adjustment process, color calibration for adjusting the color of the pickup image of the CE 11 is performed. The color calibration corrects ununiformity in color of the pickup image caused by the individual difference of each light source 30a to 30d of the light source section 30 or the CCD 25. Owing to the color calibration, the correction values for the R gain, the G gain, the B gain, the drive current value and/or light-up time of the light sources 30a to 30d, and the like are obtained.

The setting changer 46 changes various settings of the CE 11 based on a setting change command obtained through the CCD 25.

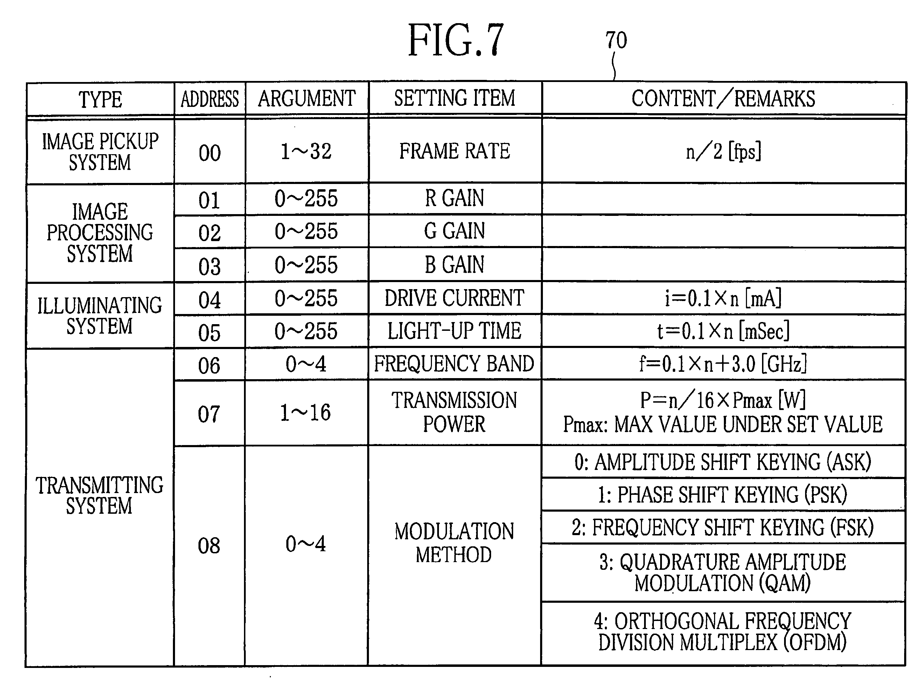

The settings of the CE 11 are described in a setting value table 70 as shown in Fig. 7. The setting value table 70 is stored in the ROM 37. The CPU 36 retrieves this setting value table 70 from the ROM 37 and controls the operations of the respective components based on the setting value table 70.

Setting items of the setting value table 70 are divided into four categories: imaging system, image processing system, illuminating system and transmitting system. The imaging system relates to the operation of the CCD 25 and the like. The image processing system relates to the operation of the signal processor 40 and the like. The illuminating system relates to the operation of the light source section 30 and the like. The transmitting system relates to the operation of the modulator 42 and the like. Each item is individually allocated with an address, and an argument corresponding to the address is stored. Before the adjustment process, standard value is set for each argument in the setting value table 70.

As the setting item of the imaging system, there is "frame rate" (address 00, argument n = 1 to 32, frame rate = n/2 [fps]) for setting the frame rate of image shooting with the CCD 25.

As the setting items of the image processing system, there are "R gain" (address 01, argument n = 0 to 255) for setting the R gain, "G gain" (address 02, argument n = 0 to 255) for setting the G gain, and "B gain" (address 03, argument n = 0 to 255) for setting the B gain.

As the setting items of the illuminating system, there are "drive current" (address 04, argument n = 0 to 255, drive current i = 0.1 × n [mA]) for setting the drive current and "light-up time" (address 05, argument n = 0 to 255, light-up time t = 0.1 × n [mSec]) for setting the light-up time.

As the setting items of the transmitting system, there are "frequency band" (address 06, argument n = 0 to 4, frequency f = 0.1 × n + 3.0 [GHz]) for setting the frequency of the radio wave 33, "transmission power" (address 07, argument n = 1 to 16, transmission power P = n / 16 × Pmax, where Pmax is a maximum value of transmission power set to be lower than the specification value) for setting the transmission power (output value) of the radio wave 33, and "modulation method" (address 08, argument n = 0 to 4, where 0 corresponds to amplitude shift keying (ASK), 1 corresponds to phase shift keying (PSK), 2 corresponds to frequency shift keying (FSK), 3 corresponds to quadrature amplitude modulation (QAM), and 4 corresponds to orthogonal frequency division multiplex (OFDM).

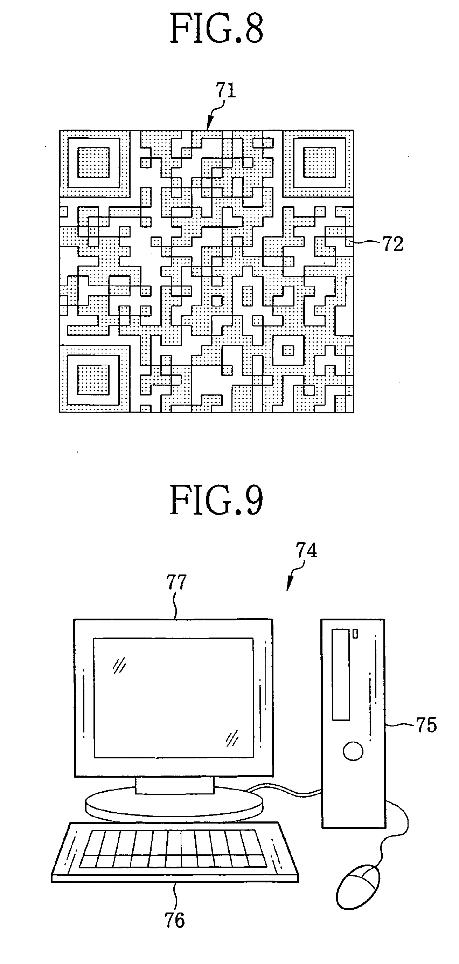

As shown in Fig. 8, the setting change command is given as a two-dimensional code (for example, QR code (trademark)) 71. In the two-dimensional code 71, as well known, cells 72 colored in black (shown with hatching) and white according to information content are two-dimensionally arranged.

The setting change command (information represented as the two-dimensional code 71) is a combination of the address and the argument corresponding to the above-described setting item. The arguments of the R gain, the G gain, the B gain, the drive current value, and the light-up time are determined based on the correction values obtained through the color calibration. The arguments of the frequency of the radio wave 33, the transmission power, and the modulation method are determined according to the destination country of the CE 11.

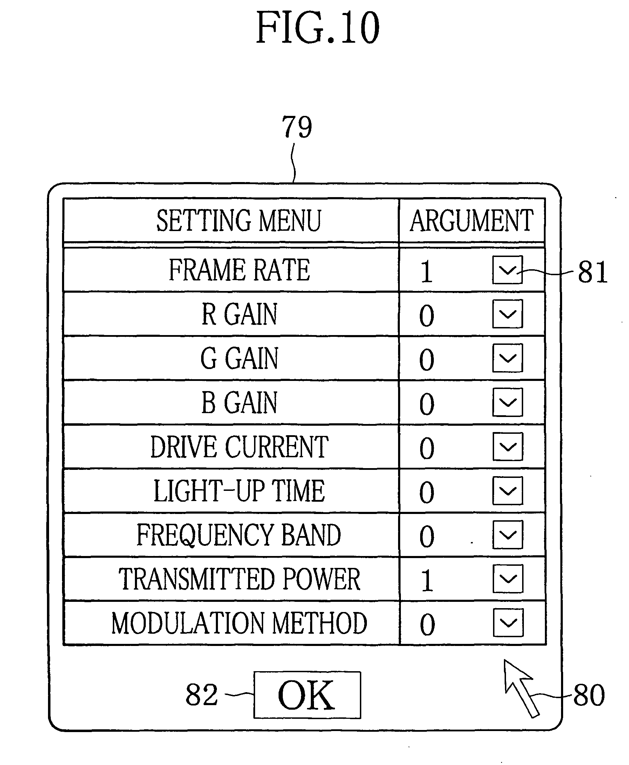

In Fig. 9, a command production device 74 includes a processor 75, a console 76 and a monitor 77. The command production device 74 is installed in a manufacturing facility of the CE 11. Programs of setting software for various settings of the CE 11 are stored in the processor 75. As the setting software is activated, a setting window 79 as shown in Fig. 10 is displayed on the monitor 77.

In the setting window 79, the setting items and their arguments are displayed. The setting of the argument can be changed upon the operation of a pull-down menu 81 using a cursor 80. When the setting of the argument is completed, an OK button 82 is selected using the cursor 80, and thereby the processor 75 produces the two-dimensional code 71 on which the setting of the argument is reflected, and the produced two-dimensional code 71 is displayed on the monitor 77.

Referring back to Fig. 5, the setting changer 46 analyzes the image data of the two-dimensional code 71 obtained with the CCD 25 and derives the setting change command from the two-dimensional code 71. The setting changer 46 rewrites the argument of the address designated by the setting change command of the item in the setting value table 70 to the value designated in the setting change command. For example, when the standard argument for the R gain is 255 and the argument designated by the setting change command is 100, the argument is rewritten to 100. When the designated argument is same as the standard argument, the argument in the setting value table 70 is not going to be overwritten. When the setting change is normally completed, the light sources 30a to 30d are lit up. Note that the setting items and the arguments are not limited to the examples shown in Fig. 7, but may appropriately be changed according to the specification of the CE 11.

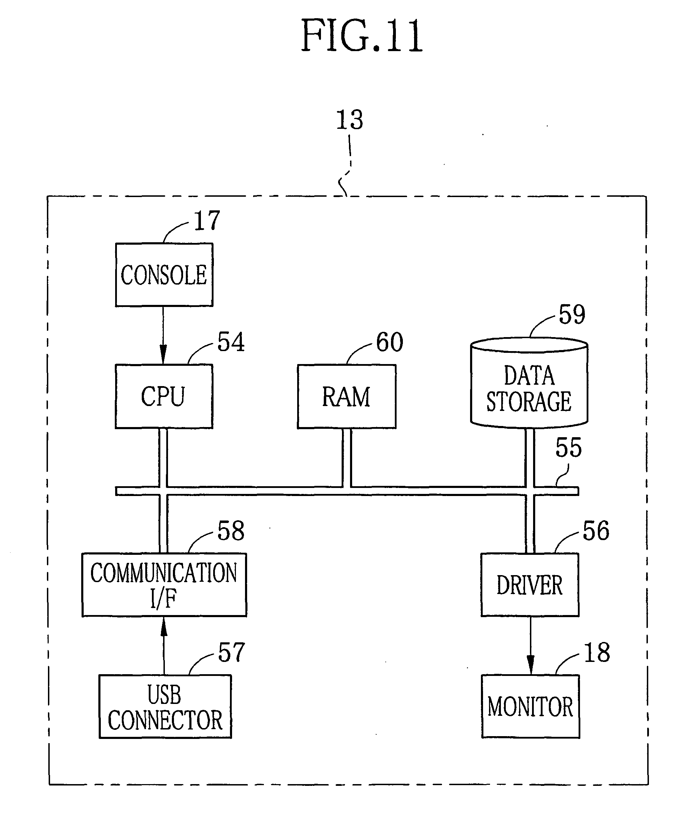

In Fig. 11, a CPU 54 controls all the operation of the WS 13 as a whole. A driver 56 for controlling the displaying of the monitor 18, a communication I/F 58 for exchanging the data with the receiver 12 via a USB connector 57 and receiving image data from the receiver 12, a data storage 59, and a RAM 60 are connected to the CPU 54 via a bus 55.

The data storage 59 stores diagnostic information for each patient together with various programs and data necessary for the operation of the WS 13 and a program of support software for supporting the doctor in the diagnosis. The RAM 60 temporarily stores the data retrieved from the data storage 59, and intermediate data generated in various kinds of arithmetic processing.

When the support software is launched, the operation window of the support software is displayed on the monitor 18, for example. The doctor can display and edit the image and input the diagnostic information by operating the console 17 on the operation window described above.

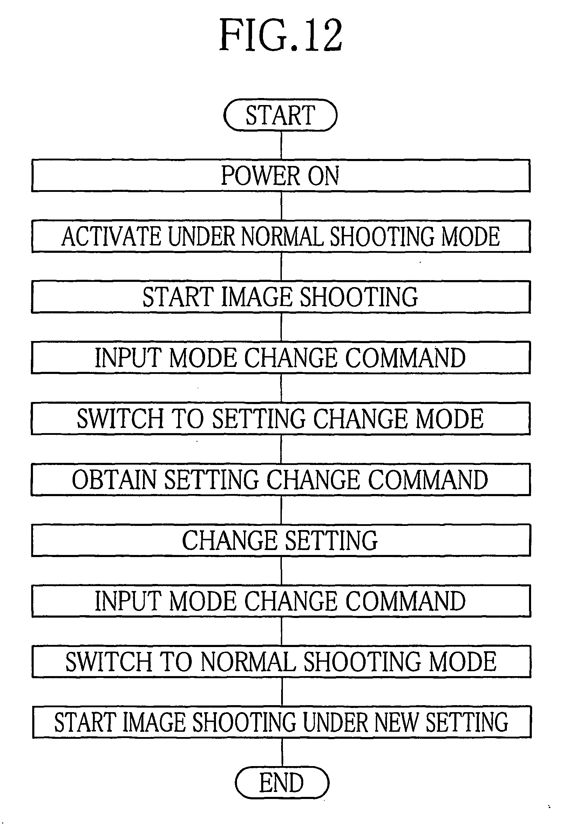

Next, the operation procedure for changing settings of the CE 11 on the above-configured capsule endoscope system 2 is explained by referring to the flowchart in Fig. 12. First of all, correction values for the R gain, G gain, B gain, drive current and light-up time are obtained by performing the color calibration. Thereafter, when the CE 11 is turned on, the program is activated and the CE 11 starts up under the normal shooting mode. The CE 11 then starts shooting images with the light sources 30a to 30d on. Moreover, the mode changer 45 and the setting changer 46 are built in the CPU 36 (see Fig. 5).

The operator operates the operation buttons 50 of the remote controller 49, and thereby emitting the mode change command from the illuminator 52. The emitted mode change command enters the CCD 25. The image data thus obtained is input to the mode changer 45. The mode changer 45 analyzes the obtained image data and switches from the normal shooting mode to the setting change mode when the image data is recognized as the mode change command.

The operator visually checks that the CE 11 is switched to the setting change mode by observing the light sources 30a to 30d turned off. The operator then operates the console 76 of the command production device 74 and activates the setting software to display the setting window 79 on the monitor 77. The operator sets the argument of each setting item on the setting window 79 by operating the console 76 based on the correction values obtained through the color calibration and the destination of the CE11 and selects the OK button 82.

When the OK button 82 is selected, the two-dimensional code 71 (see Fig. 8) on which the contents set in the setting window 79 are reflected is produced by the processor 75 and displayed on the monitor 77. The two-dimensional code 71 displayed on the monitor 77 is shot with the CCD 25. The image data of the two-dimensional code 71 thus obtained is input to the setting changer 46. Owing to this, the setting changer 46 rewrites the argument of the address designated by the setting change command of the item in the setting value table 70 to the value designated by the setting change command. The operator visually checks that the setting change is normally completed by observing the light sources 30a to 30d turned on.

After the completion of the setting change, the operator operates the operation button 50 of the remote controller 49 again, and thereby emitting the mode change command from the illuminator 52. Owing to this, the CE 11 is switched from the setting change mode to the normal shooting mode. Thereafter, the image shooting with the changed settings are performed.

As explained above, since the settings of the CE 11 can be changed by shooting the two-dimensional code 71, the setting corresponding to the specification (model) need not be performed before the assembly process in the manufacture of the CE 11. Owing to this, neither different production lines nor adjustment of the production amount depending on the specification (model) is needed, which enhances the productivity. In addition, since the CCD 25 which is certainly mounted on the CE 11 is used for changing the setting, incorporation of additional component for receiving the command is unnecessary. Moreover, since the mode changer 45 and the setting changer 46 are realized by the software, installation of additional hardware is unnecessary. Therefore, the cost increase and the enlargement of the article can be prevented, and the capacity of the battery need not be increased. Furthermore, when the CE 11 of cut-down version are mass produced and stocked, the products can be quickly delivered after merely changing the settings.

The changeover between the setting change mode and the normal shooting mode is performed based on light as the mode change command which having at least one of the color, brightness and flicker which will not appear when the inside of the body of the patient 10 is illuminated with the light source 30. Therefore, the mode is not unintentionally changed inside the body of the patient 10 during the examination, which avoids the interruption of the endoscopy.

In the above embodiment, although the mode change is performed using the mode change command emitted from the illuminator 52 of the remote controller 49, the mode change can be performed in other ways. For example, the mode can be changed using the two-dimensional code 71. Specifically, the image data of the two-dimensional code 71 obtained by the CCD 25 is analyzed in the setting changer 46 like the setting change command, and the mode change command is derived from the two-dimensional code 71. After that, the mode change is performed using the derived mode change command in the same manner as the mode change command emitted from the illuminator 52 of the remote controller 49.

It is also possible that the changeover from the normal shooting mode to the setting change mode by the mode changer 45 is performed within a certain period of time (for example, 5 minutes) after the CE 11 is turned on. That is, the mode changer 45 limits the period for receiving the mode change command to a certain time after the CE 11 is turned on and does not accept the mode change command after this period. Owing to this limitation, unintentional changeover to the setting change mode is prevented.

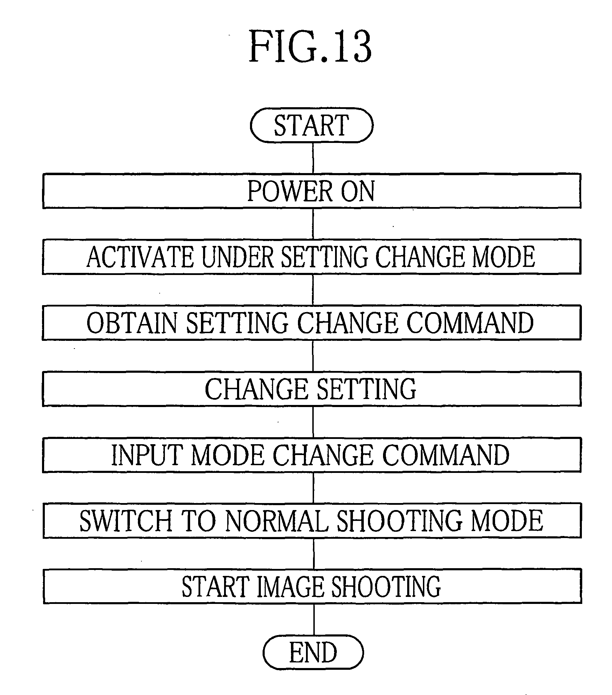

In the above embodiment, although the CE 11 starts up under the normal shooting mode when being turned on, the CE 11 may also start up under the setting change mode as shown in the flow chart of Fig. 13. In this case, when the CE 11 is turned on, the program is activated and the CE 11 starts up under the setting change mode. The mode changer 45 and the setting changer 46 are built in the CPU 35 (see Fig. 5).

The operator operates the console 76 of the command production device 74 and activates the setting software to display the setting window 79 on the monitor 77. The operator operates the console 76 to display the two-dimensional code 71 (see Fig. 8) on which the contents set in the setting window 79 are reflected on the monitor 77. The two-dimensional code 71 displayed on the monitor 77 is shot with the CCD 25 and converted into image data. The image data of the two-dimensional code 71 is input to the setting changer 46, and the argument of the address designated by the setting change command of the item in the setting value table 70 is rewritten to the value designated by the setting change command.

After checking that the setting change is normally completed by observing the light sources 30a to 30d turned on, the operator emits the mode change command to the CE 11 using the remote controller 49. Owing to this, the setting change mode is switched to the normal shooting mode and the CE 11 is turned off.

In this case, the CE 11 is set to start up under the normal shooting mode when the CE 11 is turned on again after changing the settings and once the CE 11 is turned off. Owing to this, unlike the above embodiment, the changeover from the normal shooting mode to the setting change mode is not needed. In this case, the changeover from the normal shooting mode to the setting change mode is set to be prohibited. Owing to this, the mode is not unintentionally switched inside the body of the patient 10 during the examination, which avoids the interruption of the endoscopy. The mode changeover may be performed in any manner and the trigger for the mode changeover is not limited to the form of not performing inside the body of the patient 10 (in the above embodiment, the mode change command from the remote controller 49). For example, the setting change mode can automatically be switched to the normal shooting mode after a lapse of a certain period of time (for example, 10 minutes) after the CE 11 is turned on.

In the above embodiment, although the two-dimensional code 71 is used as the setting change command, other kinds of geometric pattern such as an one-dimensional code (bar code) may be used.

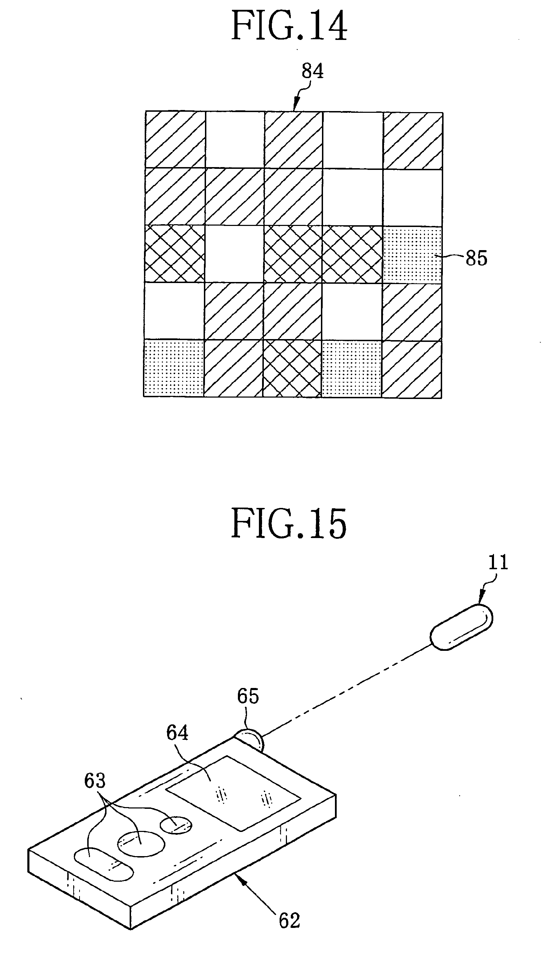

It is also possible to use a color code (color pattern) 84 shown in Fig. 14 as the setting change command. The color code 84 which is known as next generation code is composed of, for example, cells 85 arranged two-dimensionally in 5 × 5. The cells 85 are colored with four colors: red (shown with no hatching), blue (shown with cross hatching), green (shown with oblique-line hatching), and black (shown with dot hatching) according to contents of the information. The arrangement of the cells 85 is not limited to be in 5 × 5 and the shape of each cell 85 is not limited to be rectangle as long as the four colors are identifiable by, for example, integrating the area of cells 85.

It is also possible to use visible light such as white LED, infrared signal, optical signal (charges in optical intensity, optical pulse train), or the like as the setting change command. When using the infrared signal, frame rate of the CCD 25 for image shooting is raised (for example, to 1000 [fps]) so that the infrared pulse signal can be recognized. Along with the raise of the frame rate, the number of frames to be recorded in the RAM 38 increases. When the memory capacity of the RAM 38 is small, pixel skipping of the frame or partial cut out of area in the frame may be performed to reduce the data volume for each frame to the minimum extent necessary for recognizing the infrared pulse signal.

The infrared signal is, for example, generated by a remote controller 62 for infrared ray transmission as shown in Fig. 15. The remote controller 62 is constituted of operation keys 63, a display 64 for displaying operation status by the operation keys 63 and an infrared ray transmitter 65. The remote controller 62 transmits infrared ray from the infrared ray transmitter 65 when the operation key 63 is depressed. The CE 11 changes the settings when receiving the infrared ray from the remote controller 62 under the setting change mode.

In the above embodiment, although the CCD 25 is used as an example of the image sensor, the image sensor may be the CMOS. In this case, functions of the driver 39, the signal processor 40 and the like are integrally incorporated in the CMOS image sensor.

The CE 11 shown in the above embodiment is merely the example of the present invention. Various changes and modifications are possible in the present invention and may be understood to be within the present invention.

## Claims

1. A capsule endoscope (11) swallowed by a patient (10) to be inspected for shooting an image of a region to be observed inside a body of said patient, said endoscope comprising:
an illumination section (30) for emitting light to said region to be observed;
an image pickup section (25) for shooting an image of said illuminated region to be observed; and
a transmitting section (41, 42) for modulating data based on an image pickup signal obtained by said image pickup section into a radio wave (33) and transmitting said radio wave; and
**characterized by**
a setting change section (46) enclosed in the capsule endoscope (11) and adapted to change a setting of at least one of said illumination section, said image pickup section and said transmitting section based on an optically detectable setting change command obtained by said image pickup section.

2. The capsule endoscope of claim 1, wherein said setting change command is a geometric pattern.

3. The capsule endoscope of claim 2, wherein said geometric pattern is a two-dimensional code (71).

4. The capsule endoscope of claim 1, wherein said setting change command is an optical signal.

5. The capsule endoscope of claim 4, wherein said optical signal is an infrared ray signal.

6. The capsule endoscope of claim 1, wherein said setting change command is a color pattern in which predetermined areas are colored in more than one color.

7. The capsule endoscope of claim 6, wherein said color pattern is a color code (84).

8. The capsule endoscope of claim 1, wherein said data based on said image pickup signal is image pickup information.

9. The capsule endoscope of claim 1, wherein said data based on said image pickup signal is a response to said setting change command.

10. The capsule endoscope of claim 1 further comprising:
a mode change section (45) for switching between a setting change mode for receiving said setting change command and a normal shooting mode for shooting said region to be observed, based on a mode change command obtained by said image pickup section.

11. The capsule endoscope of claim 10, wherein said mode change command is light including at least one of color, brightness and flicker not appearing when said region to be observed is illuminated with said illumination section.

12. The capsule endoscope of claim 10, wherein said mode change section switches from said normal shooting mode to said setting change mode within a certain period of time after a power is turned on.

13. The capsule endoscope of claim 1 further comprising:
a mode change section for activating under a setting change mode for receiving said setting change command when a power is turned on, and switching from said setting change mode to a normal shooting mode for shooting said region to be observed when a predetermined condition is satisfied.

14. The capsule endoscope of claim 13, wherein said setting change section and said mode change section are realized by software.

15. The capsule endoscope of claim 14, wherein said setting change command sets drive condition of said illumination section.

16. The capsule endoscope of claim 15, wherein said drive condition includes at least one of a drive current value and a light-up time of a light source constituting said illumination section.

17. The capsule endoscope of claim 1, wherein said setting change command sets a frame rate for image shooting by said image pickup section.

18. The capsule endoscope of claim 17, wherein said setting change command sets at least one of a transmit frequency, a modulation method and an output value of said transmitting section.

19. A capsule endoscope system (2) comprising:
(A) a capsule endoscope (11), including:
an illumination section (30) for emitting light to a region to be observed inside a body of a patient (10);
an image pickup section (25) for shooting an image of said illuminated region to be observed;
a transmitting section (41, 42) for modulating data based on an image pickup signal obtained by said image pickup section into a radio wave (33) and transmitting said radio wave; and
a setting change section (46) enclosed in the capsule endoscope and adapted to change a setting of at least one of said illumination section, said image pickup section and said transmitting section based an optically detectable setting change command obtained by said image pickup section; and
(B) a command production device (74) for producing said setting change command.

## Patentansprüche

1. Kapselendoskop (11), welches von einem zu untersuchenden Patienten (10) zu schlucken ist, um ein Bild einer Betrachtungszone im Inneren des Körpers des Patienten aufzunehmen, wobei das Endoskop aufweist:
einen Beleuchtungsabschnitt (30) zumEmittieren von Licht auf die Betrachtungszone;
einen Bildaufnahmeabschnitt (25) zum Aufnehmen eines Bildes der beleuchteten Betrachtungszone;
einen Sendeabschnitt (41, 42) zum Modulieren von Daten basierend auf dem von dem Bildaufnahmeabschnitt erhaltenen Bildaufnahmesignal zu einer Funkwelle (33) und zum Senden der Funkwelle;
**gekennzeichnet durch**
einen Einstellungsänderungsabschnitt (46), der in dem Kapselendoskop (11) eingeschlossen und dazu ausgebildet ist, eine Einstellung mindestens eines Abschnitts von dem Beleuchtungsabschnitt, dem Bildaufnahmeabschnitt und dem Sendeabschnitt basierend auf einem optisch nachweisbaren Einstellungs-Änderungsbefehl, der von dem Bildaufnahmeabschnitt erhalten wird, zu ändern.

2. Kapselendoskop nach Anspruch 1, bei dem der Einstellungs-Änderungsbefehl ein geometrisches Muster ist.

3. Kapselendoskop nach Anspruch 2, bei dem das geometrische Muster ein zweidimensionaler Code (71) ist.

4. Kapselendoskop nach Anspruch 1, bei dem der Einstellungs-Änderungsbefehl ein optisches Signal ist.

5. Kapselendoskop nach Anspruch 4, bei dem das optische Signal ein Infrarotstrahl-Signal ist.

6. Kapselendoskop nach Anspruch 1, bei dem der Einstellungs-Änderungsbefehl ein Farbmuster ist, in welchem vorbestimmte Flächen in mehr als einer Farbe gefärbt sind.

7. Kapselendoskop nach Anspruch 6, bei dem das Farbmuster ein Farbcode (84) ist.

8. Kapselendoskop nach Anspruch 1, bei dem die auf dem Bildaufnahmesignal basierenden Daten Bildaufnahmeinformation sind.

9. Kapselendoskop nach Anspruch 1, bei dem die auf dem Bildaufnahmesignal basierenden Daten eine Antwort auf den Einstellungs-Änderungsbefehl sind.

10. Kapselendoskop nach Anspruch 1, weiterhin umfassend:
einen Modusänderungsabschnitt (45) zum Umschalten zwischen einem Einstellungs-Änderungsmodus zum Empfangen des Einstellungs-Änderungsbefehls und einem Normalaufnahmemodus zum Aufnehmen der Betrachtunszone, basierend auf einem von dem Bildaufnahmeabschnitt erhaltenen Modus-Änderungsbefehl.

11. Kapselendoskop nach Anspruch 10, bei dem der Modus-Änderungsbefehl Licht ist, das mindestens eine Farbe, eine Helligkeit und/oder ein Flimmern enthält, welches dann nicht erscheint, wenn die Betrachtungszonen mit dem Beleuchtungsabschnitt beleuchtet werden.

12. Kapselendoskop nach Anspruch 10, bei dem der Modus-Änderungsabschnitt umschaltet von dem Normalaufnahmemodus in Einstellungs-Änderungsmodus innerhalb einer gewissen Zeitspanne nach Einschalten der Stromversorgung.

13. Kapselendoskop nach Anspruch 1, weiterhin umfassend:
einen Modus- Änderungsabschnitt zum Aktivieren unter einem Einstellungs-Änderungsmodus zum Empfangen des Einstellungs-Änderungsbefehls, wenn die Stromversorgung eingeschaltet wird, und zum Umschalten aus dem Einstellungs-Änderungsmodus in einen Normalaufnahmemodus zum Aufnehmen der Betrachtungszone, wenn eine vorbestimmte Bedingung erfüllt ist.

14. Kapselendoskop nach Anspruch 13, bei dem der Einstellungs-Änderungsabschnitt und der Modus- Änderungsabschnitt durch Software realisiert sind.

15. Kapselendoskop nach Anspruch 14, bei dem der Einstellungs-Änderungsbefehl eine Treiberbedingung für den Beleuchtungsabschnitt einstellt.

16. Kapseledoskop nach Anspruch 15, bei dem die Treiberbedingung mindestens einen von einem Treiberstromwert und/oder einer Einschaltzeit eine den Beleuchtungsabschnitt bildenden Lichtquelle beinhaltet.

17. Kapselendoskop nach Anspruch 1, bei dem der Einstellungs-Änderungsbefehl eine Vollbildrate für die Bildaufnahme durch den Bildaufnahmeabschnitt einstellt.

18. Kapselendoskop nach Anspruch 17, bei dem der Einstellungs-Änderungsbefehl mindestens eine Sendefrequenz, ein Modulationsverfahren und/oder einen Ausgangswert des Sendeabschnitts einstellt.

19. Kapselendoskopsystem (2), umfassend:
(A) ein Kapselendoskop (11), enthaltend:
einen Beleuchtungsabschnitt (30) zum Emittieren von Licht auf eine Betrachtungszone im Inneren eines Körpers eines Patienten (10);
einen Bildaufnahmeabschnitt (25) zum Aufnehmen eines Bildes der beleuchteten Betrachtungszone;
einen Sendeabschnitt (41, 42) zum Modulieren von Daten basierend auf einem von dem Bildaufnahmeabschnitt erhaltenen Bildaufnahmesignal in eine Funkwelle (33), und Senden der Funkwelle; und
einen Einstellungs-Änderungsabschnitt (46), der in dem Kapselendoskop eingeschlossen und dazu ausgebildet ist, eine Einstellung mindestens des Beleuchtungsabschnitts, des Bildaufnahmeabschnitts, und/oder des Sendeabschnitts basierend auf einem optisch nachweisbaren Einstellungs-Änderungsbefehl, der von dem Bildaufnahmeabschnitt erhalten wird, zu ändern; und
(B) eine Befehlserzeugungseinrichtung (74) zum Erzeugen des Einstellungs-Änderungsbefehls.

## Revendications

1. Endoscope à capsule (11) avalé par un patient (10) à examiner destiné à prendre une image d'une région devant être observée à l'intérieur du corps dudit patient, ledit endoscope comprenant :
une section d'éclairage (30) destinée à émettre de la lumière vers ladite région à observer ;
une section de capture d'image (25) destinée à prendre une image de ladite région éclairée à observer ; et
une section de transmission (41, 42) destinée à moduler des données sur la base d'un signal de capture d'image obtenu par ladite section de capture d'image en une onde radio (33) et à transmettre ladite onde radio ; et
**caractérisé par** une section de changement de réglage (46) enfermée dans l'endoscope à capsule (11) et conçue pour changer un réglage d'au moins une section parmi ladite section d'éclairage, ladite section de capture d'image et ladite section de transmission sur la base d'une commande de changement de réglage optiquement détectable obtenue par ladite section de capture d'image.

2. Endoscope à capsule selon la revendication 1, dans lequel ladite commande de changement de réglage est un tracé géométrique.

3. Endoscope à capsule selon la revendication 2, dans lequel ledit tracé géométrique est un code bidimensionnel (71).

4. Endoscope à capsule selon la revendication 1, dans lequel ladite commande de changement de réglage est un signal optique.

5. Endoscope à capsule selon la revendication 4, dans lequel ledit signal optique est un signal à rayons infrarouges.

6. Endoscope à capsule selon la revendication 1, dans lequel ladite commande de changement de réglage est un tracé en couleur dans lequel des zones prédéfinies sont colorées en plus d'une couleur.

7. Endoscope à capsule selon la revendication 6, dans lequel ledit tracé en couleur est un code couleur (84).

8. Endoscope à capsule selon la revendication 1, dans lequel lesdites données basées sur ledit signal de capture d'image sont des informations de capture d'image.

9. Endoscope à capsule selon la revendication 1, dans lequel lesdites données basées sur ledit signal de capture d'image sont une réponse à ladite commande de changement de réglage.

10. Endoscope à capsule selon la revendication 1 comprenant en outre :
une section de changement de mode (45) destinée à alterner entre un mode changement de réglage servant à recevoir ladite commande de changement de réglage et un mode prise de vue normal servant à prendre en photo ladite région à observer, sur la base d'une commande de changement de mode obtenue par ladite section de capture d'image.

11. Endoscope à capsule selon la revendication 10, dans lequel ladite commande de changement de mode est une lumière comprenant au moins une caractéristique parmi une couleur, une luminosité et un vacillement n'apparaissant pas lorsque ladite région à observer est éclairée à l'aide de ladite section d'éclairage.

12. Endoscope à capsule selon la revendication 10, dans lequel ladite section de changement de mode passe dudit mode prise de vue normal audit mode changement de réglage dans une certaine période après avoir été mise sous tension.

13. Endoscope à capsule selon la revendication 1 comprenant en outre :
une section de changement de mode destinée à s'activer dans un mode changement de réglage servant à recevoir ladite commande de changement de réglage lorsqu'elle est sous tension, et à passer dudit mode changement de réglage à un mode prise de vue normal servant à prendre en photo ladite région à observer lorsqu'une condition prédéfinie est satisfaite.

14. Endoscope à capsule selon la revendication 13, dans lequel ladite section de changement de réglage et ladite section de changement de mode sont réalisées par logiciel.

15. Endoscope à capsule selon la revendication 14, dans lequel ladite commande de changement de réglage règle la condition d'entraînement de ladite section d'éclairage.

16. Endoscope à capsule selon la revendication 15, dans lequel ladite condition d'entraînement comprend au moins une caractéristique parmi une valeur de courant d'entraînement et un temps d'allumage d'une source de lumière constituant ladite section d'éclairage.

17. Endoscope à capsule selon la revendication 1, dans lequel ladite commande de changement de réglage règle une cadence de prise de vue pour prise d'image par ladite section de capture d'image.

18. Endoscope à capsule selon la revendication 17, dans lequel ladite commande de changement de réglage règle au moins une caractéristique parmi une fréquence de transmission, un procédé de modulation et une valeur de sortie de ladite section de transmission.

19. Système d'endoscope à capsule (2) comprenant :
(A) un endoscope à capsule (11), comprenant :
une section d'éclairage (30) destinée à émettre de la lumière vers une région devant être observée à l'intérieur du corps d'un patient (10) ;
une section de capture d'image (25) destinée à prendre une image de ladite région éclairée à observer ;
une section de transmission (41, 42) destinée à moduler des données sur la base d'un signal de capture d'image obtenu par ladite section de capture d'image en une onde radio (33) et à transmettre ladite onde radio ; et
une section de changement de réglage (46) enfermée dans l'endoscope à capsule et conçue pour changer un réglage d'au moins une section parmi ladite section d'éclairage, ladite section de capture d'image et ladite section de transmission sur la base d'une commande de changement de réglage optiquement détectable obtenue par ladite section de capture d'image ; et
(B) un dispositif de production de commande (74) destiné à produire ladite commande de changement de réglage.
